# EUROPEAN PATENT APPLICATION

(11) **EP 1 494 029 A1**
(43) Date of publication of application: **05.01.2005**
(21) Application number: 03746408.8
(22) Date of filing: 28.03.2003
(51) Int. Cl.: G01N 33/543, G01N 37/00

(54) **METHOD OF ANALYZING MOLECULE AND MOLECULE ANALYZER**

(30) Priority: 16.04.2002 JP 2002112937
(71) Applicant: Nano Solution, Inc., Setagaya-ku, Tokyo 154-0012 (JP)
(72) Inventor: NATSUME, Tohru, Meguro-ku, Tokyo 153-0063 (JP)
(74) Representative: Leson, Thomas Johannes Alois, Dipl.-Ing.
(86) International application number: PCT/JP2003/003943
(87) International publication number: WO 2003/087826

(57) **Abstract**

Provided is a method of analyzing molecule comprising at least a molecule transfer step in which target molecules having shown interactions with detection molecules, which are previously bound to a detection surface (of a sensor chip) of a biosensor for analyzing the intermolecular interactions, are compressed to a solid phase such as a membrane instead of a liquid phase to be transferred thereonto, and recovered, thus making an operation for analyzing a structure and the like of the target molecules captured by the biosensor easy and achieving high analysis precision. Furthermore, a molecule analyzer is provided, which is contrived so as to be capable of automatically carrying out the method of analyzing molecule, and which can exhibit the same effects as those of the method of analyzing molecule.

## Description

### Field of the Invention

The present invention relates to an analysis technology useful in fields of biochemistry, molecular biology and the like. More specifically, the present invention relates to a molecule analysis technology especially useful for analyzing a sequence and structure of a target molecule showing a interaction with a detection molecule which is previously immobilized on a detection surface area of a sensor chip of a biosensor.

### Background Technology

An analyzer called a biosensor in which a sensor chip having a detection surface area is incorporated has currently been spread.

The biosensor is a useful tool for analyzing intermolecular interactions in amino acids, peptides, proteins, nucleotides, low molecular compounds, lipids and the like, including antigen-antibody reaction, hybridization and enzyme response. The biosensor is also a useful tool for kinetic analysis of these intermolecular interactions.

Herein, the detection surface area of the sensor chip is previously undergone a treatment by which substances having functional groups suitable for immobilizing detection molecules, for example, carboxyl group, amino group, aldehyide group, thiol group and the like, and streptavidin are immobilized thereon.

If target molecules exist in a sample solution supplied on a detection surface, the detection molecules bound to the detection surface area to be immobilized thereon show specific interactions to capture the target molecules.

The biosensor performs real-time detection of a binding/dissociation state of the interaction by use of principles such as surface plasma resonance and quartz-crystal microbalance.

In recent years, a method has been devised, in which after interactions are performed on a detection surface, target molecules captured by the detection molecules are recovered by a specified solution of a very small volume, and a further detailed molecule analysis, for example, mass spectrometry, is performed. In mass spectrometry, captured target molecules are recovered, and a sample solution containing the target molecules to be subjected to mass spectrometry is prepared.

Since precision of the molecule analysis using a mass spectrometer, which is subsequent to the biosensor, depends largely on the sample concentration in the sample solution, it is essential to recover the target molecules by as a small amount of recovery solution as possible.

However, a highly skilled technique has been required for preparation and solution-sending operations of a trace amount of the recovery solution. Furthermore, since the recovery operation by use of this solution takes much time, an improvement to enhance operation efficiency by realizing high throughput has been tried. However, there has been a technical problem that the enhancement of the operation efficiency is limited, which is difficult to solve.

Accordingly, an object of the present invention is to provide a technology to transfer target molecules, which are captured on a detection surface of a sensor chip, on a membrane, and to recover them. Furthermore, an object of the present invention is to provide a method of analyzing molecule and a molecule analyzer, which use this technology.

### Disclosure of the Invention

To solve the above described technical problem, this application provides a method of analyzing molecule which is devised so as to comprising at least a molecule transfer step for transferring target molecules onto a solid phase, the target molecules showing interactions with detection molecules bound to a detection surface of a biosensor for analyzing intermolecular interactions.

To be concrete, after completion of the intermolecular interactions on the detection surface formed in the sensor chip of the biosensor, the detection surface portion is detached from a sensor unit portion, and the target molecules are transferred onto the solid phase.

Herein, the word "transfer" widely means a step of allowing molecules captured on a detection surface to migrate to a solid phase that is a material for transfer, or step of transferring the molecules. The word "transfer" is not interpreted narrowly. With respect to a molecule transfer step, one step selected among an electrical transfer step, a compression transfer step and an aspiration transfer step can be adopted, and these steps may be combined properly.

The word "detection surface" means a surface area which is provided in the sensor chip disposed in the sensor unit portion which performs interactions detection by means of, for example, plasmon resonance or quartz-crystal microbalance, and which has been subjected to a treatment so as to be capable of intermolecular interactions. When the method according to the present invention is carried out, it is preferable to adopt a structure that the sensor chip can be attached/detached as easily as possible in the sensor unit portion.

The method of analyzing molecule according to the present invention has a technological concept largely shifted from conventional and extremely generalized idea that molecules captured on the detection surface are recovered in a liquid phase, and the molecule analysis according to the present invention has a theme to provide a perfectly novel molecule recovery technology in which the target molecules captured on the detection surface of the chip of the biosensor are transferred onto a solid phase, most notably a membrane to be recovered.

The word "solid phase" includes a solid body and a semi-solid body having a property to be able of accepting the target molecule captured on the detection surface to retain them.

The word "membrane" is a porous material having a liquid permeability, and as the membrane, polyvinyl alcohol (PVA) membrane, a nylon membrane, a nitrocellulose membrane and a membrane filter can be enumerated.

The reason why the membrane is suitable as the solid phase that is the transfer object is that an post-treatment such as wash and the like can be performed suitably because the membrane is easily brought into an intimate contact with the detection surface owing to its flexibility and possess a liquid permeability.

If the foregoing molecule transfer step is adopted, even a beginner can recover the target molecules captured on the detected surface without contamination of the target molecules, and time required for a molecule recovery operation can be also shortened greatly. Furthermore, a preparation operation for a recovery column, which is a troublesome task, is unnecessary.

Since it is unnecessary to perform a recovery operation for the detection molecules by use of a solution, a problem burdened with a conventional liquid phase recovery method can be solved radically, in which taints existing in a flow channel is mixed into a very small amount of sample solution, and the taints become an obstacle to the analysis.

Furthermore, in recent years, a technology may be tried, in which the detection surface of the sensor chip is partitioned, a plural species of detection molecules are individually immobilized, and the target molecules showing interactions with the respective detection molecules are detected and analyzed.

In this case, if the molecule transfer step of the present invention is adopted, there is a merit that the plural species of detection molecules can be separately and simultaneously transferred, recovered and analyzed without mixing them with certainty.

To be concrete, in the conventional recovery step by use of the solution, since the recovery operations for the target molecules captured by the respective detection molecules on the detection surface has had to be done sequentially under individually independent operations by use of flow channels partitioned, a lot of trouble and time have been taken. During the recovery operation for a long period of time, the target molecules may dissociate and denature.

On the other hand, since in the molecule transfer step in the present invention, the plural species of the target molecules captured on the detection surface can be transferred and recovered at a time, a lot of trouble is not necessary, and the recovery operation can be completed in a short time. Furthermore, since much time is not required for the recovery operation, no problem that the target molecules dissociate and denature occurs.

Herein, a concrete example of a series of steps of the method of analyzing molecule according to the present invention is presented. Specifically, mentioned is a step in which a solution containing target molecules is allowed to contact a detection surface of a biosensor for analyzing intermolecular interactions, the target molecules, which have reacted with detection molecules actively, captured on the detection surface are subsequently transferred onto a membrane, and the target molecules transferred onto the membrane are eluted into a liquid phase by use of, for example, enzyme digestion, followed by analyzing a sample solution by mass spectrometry.

Note that in mass spectrometry, it is possible to identify molecular species of the target molecules, and to perform a structural analysis. For example, when the target molecule is protein, it is possible to perform analyses of the amino acid sequence, states of phospholylation and glycosylation, and when the target molecule is lipid, it is possible to perform an analysis of side chain length.

Next, in addition to the foregoing method of analyzing molecule, the present invention provides a molecule analyzer comprising at least automatic molecule transfer means which is devised so as to be capable of automatically transferring target molecules onto a membrane, the target molecules having shown interactions with detection molecules bound to a detection surface of a biosensor for analyzing intermolecular interactions.

Specifically, after completion of an interaction step on the detection surface, a sensor chip or a detection surface area is taken out from the biosensor, and the sensor chip or the detection surface area is set up at a predetermined spot. Subsequently, by one means selected from an electrical transfer, a compression transfer and an aspiration transfer, the detection molecules are automatically transferred onto a solid phase such as a membrane previously set up at a position opposite to the sensor chip.

It should be noted that in this apparatus, means for capable of automatically executing steps of wash, modification, enzyme digestion and extraction, which are useful for a subsequent analysis, may be provided in addition to the transfer means.

As described above, the method of analyzing molecule and the molecule analyzer according to the present invention have technological significance that by recovering the molecules, captured on the detection surface of the biosensor, onto the solid phase, operating efficiency can be improved, and precision of a subsequent molecule analysis can be increased.

### Brief Description of the Drawings

Fig. 1 is a diagram briefly showing concepts of molecule transfer step of a method of analyzing molecule and molecule transfer means of a molecule analyzer according to the present invention.
Fig. 2 is a diagram briefly showing a state of a membrane (2) after a molecule transfer operation.
Fig. 3 is a block diagram briefly showing a fundamental concept of the molecule analyzer according to the present invention.
Fig. 4 is a diagram (graph) showing a real-time/response curve by surface plasmon resonance.
Fig. 5 is a diagram (photograph) showing a transfer state onto a membrane.

### Best Mode for Carrying Out the Invention

A preferred embodiment of a method of analyzing molecule and a molecule analyzer according to the present invention will be described with reference to the accompanying drawings below.

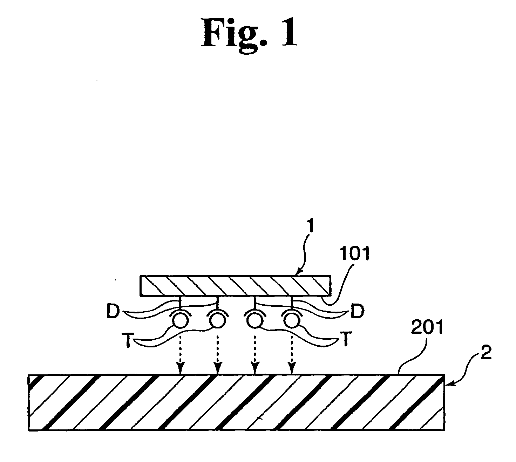

First, Fig. 1 is a diagram briefly showing concepts of molecule transfer step of a method of analyzing molecule and molecule transfer means of a molecule analyzer according to the present invention.

Reference numeral 1 in Fig, 1 denotes a detection surface of a sensor chip detached from a sensor portion of a biosensor after intermolecular interactions using the biosensor.

Detection molecules D are previously immobilized on a reaction plane 101 of the detection surface 1, and target molecules T showing interactions actively with the detection molecules D are bound to the molecules D by the intermolecular interactions. It should be noted that in the present invention, a type of the biosensor and a structure of the detection surface are selectable suitably and never limited narrowly.

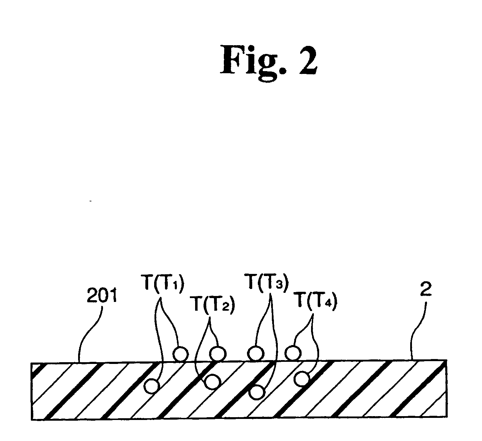

Reference numeral 2 in Fig. 2 denotes a membrane such as a PVA membrane, a nylon membrane, a nitroglycerin membrane and a membrane filter. The reaction plane 101 of the detection surface 1 is allowed to be opposite to the membrane 2, and thereafter a state where the reaction plane 101 and a transfer plane 201 of the membrane 2 are in contact with each other is ensured. Subsequently, the target molecules T are migrated to the transfer plane 201 of the membrane 2 from the reaction plane 101 of the detection surface 1 by applying electricity to the detection surface 1, compressing the detection surface 1 to the transfer plane 201 to press-fit the surface 1 to the plane 201, and utilizing suction such as vacuum suction (molecular transfer step).

Fig. 2 is a diagram briefly showing a state of the membrane 2 after the molecule transfer operation. As shown in Fig. 2, after completion of the molecule transfer operation, the molecules T migrate from the detection surface 1 to the transfer plane 201 of the membrane 2, and the target molecules T enter a state where they adhere to the transfer plane 201. Specifically, the target molecules T having shown interactions with the detection molecules D are recovered in a solid phase certainly

When a plural species of the target molecules T₁, T₂, T₃ and T₄ are captured on the detection surface 1, these target molecules T₁, T₂, T₃ and T₄ are transferred onto the transfer plane 201 of the membrane 2 by one molecule transfer operation.

Although also the detection molecules D may be simultaneously transferred onto the membrane 2 in addition to the target molecules T, in this case, it is possible to discriminate both of the molecules T and D from each other by an subsequent analysis. Therefore, there is no problem in analyzing a structure and the like of the target molecules T.

The target molecules which have been transferred and recovered on the membrane 2 are subjected to treatments such as wash, modification, enzyme digestion and extraction, which are selectively necessary depending on the subsequent analysis method, and identification of molecular species, structural analysis and the like are performed.

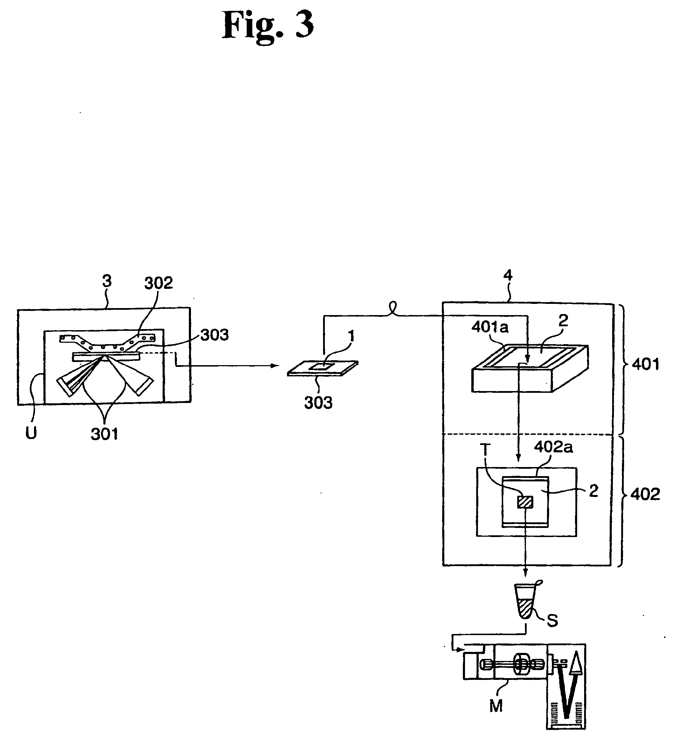

Next, a constitution of the molecule analyzer according to the present invention will be described based on Fig. 3 attached. Fig. 3 is a block diagram briefly showing a fundamental concept of the molecule analyzer according to the present invention.

Reference numeral 3 in Fig. 3 represents a principal part of a biosensor using surface plasmon resonance (SPR) which is an example of the biosensor, and a sensor unit portion indicated by reference symbol U is provided in the biosensor 3.

This sensor unit portion U comprises SPR detection means 301 for optically detecting surface plasmon resonance occurred when surface plasmon is excited at a metal/liquid interface.

The SPR detection means 301 detects, as an SPR signal, a subtle mass change which occurs at the detected surface of a sensor chip in conjunction with binding and dissociation of the detection molecules and the target molecules.

Furthermore, the sensor unit portion U generally comprises a micro flow channel 302 for performing a precise control of solution supply to the detection surface 1, and a sensor chip 303.

The detection surface 1 is formed at a predetermined spot of the sensor chip 303. The detection surface 1 has, for example, a constitution in which a gold thin film is evaporated on a glass substrate, and offers a place where the detection molecules D immobilized on the gold thin film side and the target molecules T contained in a solution sent by the controlled micro flow channel perform interactions.

It should be noted that in the sensor unit portion U, a cuvette system may be adopted instead of the micro flow channel 302.

In the biosensor 3 constituted as above, after the intermolecular interactions between the molecules D and T are performed in accordance with the predetermined step, the sensor chip 303 is detached from the apparatus 3, and transported to an automatic molecule transfer apparatus 4.

Subsequently, the sensor chip 303 or the detection surface 1 detached from the sensor chip 303 is set up at a predetermined transfer spot 401a of an automatic transfer portion 401. It should be noted that a set-up step of setting up the sensor chip 303 to the transfer spot 401a may be automated.

The membrane 2 is previously set up at the foregoing transfer spot 401a, and the foregoing sensor chip 303 is set up so that the detection surface 1 thereof faces the foregoing membrane 2.

The transfer can be satisfactorily performed by any means suitably selected from electric transfer, compression transfer and aspiration transfer. It should be noted that transfer means in which these steps are combined may be adopted. For example, the transfer spot 401a is allowed to enter a state where the transfer spot 401a is hermetically sealed, and means for aspiration and compression may be adopted.

Subsequently, after completion of the transfer operation, the membrane 2 is taken out from the transfer spot 401a, and is transported to a predetermined spot 402a of an analysis sample preparation portion 402. Each step from taking-out of the membrane 2 from the transfer spot 401a to the transportation of the membrane 401a to the analysis sample preparation portion 402 may be automated.

The analysis sample preparation portion 402 is configured to be capable of suitably selecting the step of wash, modification, enzyme digestion and extraction, which are pretreatment steps to obtain a sample S suitable for a subsequent molecule analysis, and these steps can be carried out automatically.

In Fig. 3, a constitution in which the automatic transfer portion 401 and the analysis sample preparation portion 402 are partitioned is exemplified. The automatic transfer portion 401 and the analysis sample preparation portion 402 are integrated, and the transfer and the pretreatment step may be continuously performed in the automatic transfer portion 401 without transporting the membrane 2.

The sample S obtained by the pretreatment in the analysis sample preparation portion 402 is put through a molecule analyzer M such as a mass spectrometer, and identification of molecular species and a structural analysis of the molecules are carried out.

### <Example>

The inventor of the present application carried out a verification examination as to whether or not the target molecules captured on the detection surface of the sensor chip are transferred onto the membrane.

### Experiment Conditions and Experiment Method

As the biosensor, a surface plasmon resonance sensor (BIACORE3000), made by Bia Core Co. Ltd., was used, and as the sensor chip, CM5, made by Bia Core Co. Ltd., was used.

As the detection molecule, anti Glutathione S-transferase (GST) antibody was immobilized on the sensor chip surface by amine coupling. Thereafter, GST (that is the target molecule) is supplied onto the surface of the sensor chip, and is allowed to react with the anti GST antibody.

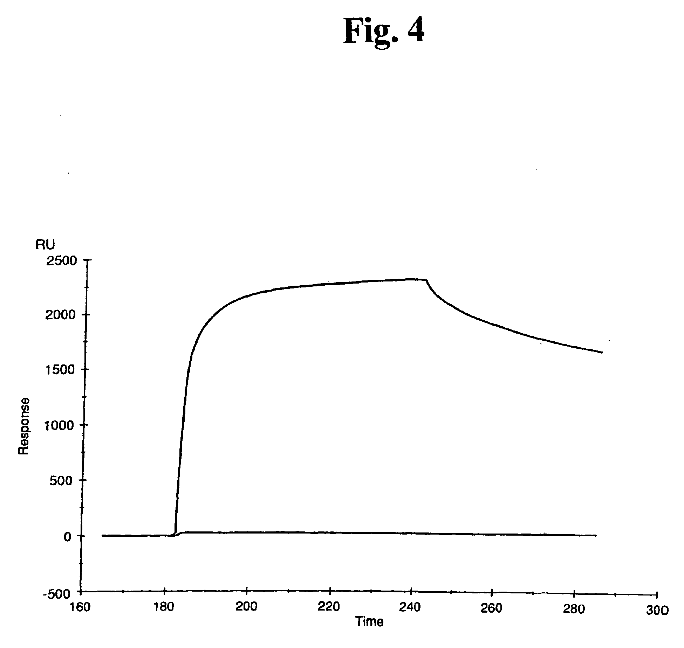

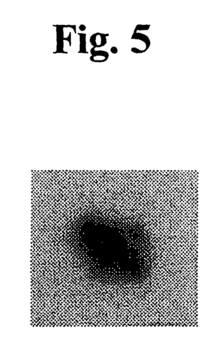

During that time, a real-time-/reaction curve by the surface plasmon resonace illustrated in Fig. 4 was observed, and it was confirmed that a sufficient amount of binding was obtained. The sensor chip was detached from the biosensor, and a polyvinyl alcohol transfer membrane (PBA membrane) was compressed to the surface of the detached sensor chip for ten minutes. A state where the PVA membrane was compressed to the surface of the sensor chip is shown in Fig. 5 (photograph).

The transfer efficiency was evaluated by Western blotting. In the Western blotting, the same anti GST antibody as that used as the detection molecules was used.

### Experiment Result and Consideration

From the real-time/response curve by the surface plasmon resonance, it was assumed that about 2 nano grams of the target molecules are bound on the sensor chip. When the PVA membrane was compressed to the surface of the sensor chip and the transfer operation was performed, a signal of the target molecules was detected from the membrane very significantly.

This means that any given target molecules bound on the sensor chip were recovered on the membrane with high efficiency, and that it is possible to analyze the bound molecules by mass spectrometry and the like with high sensitivity.

### Industrial Applicability

According to the method of analyzing molecule or the molecule analyzer according to the present invention, even a beginner can recover the target molecules, captured on the detection surface, on the solid phase simply, so that time required for the molecule recovering operation can be shortened greatly. Remarkable convenience is brought about. A troublesome preparation operation of a recovery column is unnecessary. In addition, since it is unnecessary to perform a recovery operation for the detection molecules by use of a solution, taints existing the flow channel of the biosensor are never mixed into a very small amount of sample solution to become an obstacle to the analysis. Accordingly, a molecule analysis precision can be increased.

When the plural species of detection molecules are immobilized individually and the target molecules having shown the interactions with the respective detection molecules are detected and analyzed, one molecule transfer step or molecule transfer means is executed, whereby the plural species of target molecules can be transferred, recovered and analyzed quickly and certainly.

The method of analyzing molecule or the molecule analyzer according to the present invention can greatly increase the operation efficiency related to the molecule analysis, as a result of contriving to recover the molecules, captured in the detection surface of the biosensor, on the solid phase, and can enhance the subsequent molecule analysis precision.

## Claims

1. A method of analyzing molecule comprising:
at least a molecule transfer step of transferring target molecules onto a solid phase, the target molecules having shown interactions with detection molecules bound to a detection surface of a biosensor for analyzing intermolecular interactions.

2. The method according to claim 1, wherein the solid phase is a membrane.

3. The method according to one of claims 1 and 2, wherein the molecule transfer step includes at least one step selected from an electrical transfer, a compression transfer and an aspiration transfer.

4. The method according to claim 1, wherein the detection surface is one disposed in a sensor unit portion for detecting the interactions by plasmon resonance or quartz-crystal microbalance.

5. The method according to claim 1, wherein the target molecules are analyzed by mass spectrometry, the target molecules being bound to the detection surface of the biosensor for analyzing the intermolecular interactions recovered on a membrane by the molecule transfer step.

6. A molecule analyzer, comprising:
at least automatic molecule transfer means for automatically transferring target molecules onto a solid phase, the target molecule having shown interactions with detection molecules bound to a detection surface of a biosensor for analyzing intermolecular interactions.

7. The molecule analyzer according to claim 6, wherein the solid phase is a membrane.

8. The molecule analyzer according to one of claims 6 and 7, wherein the molecule transfer means includes at least one means selected from an electrical transfer, a compression transfer and an aspiration transfer.
